# EUROPEAN PATENT APPLICATION

(11) **EP 4 650 450 A1**
(43) Date of publication of application: **19.11.2025**
(21) Application number: 23915192.1
(22) Date of filing: 16.11.2023
(51) Int. Cl.: C12N 15/13, A61K 39/395, A61P 35/00, C07K 16/28, C12N 1/15, C12N 1/19, C12N 1/21, C12N 5/10, C12N 15/63

(54) **FELINE ANTI-PD-1 ANTIBODY**

(30) Priority: 12.01.2023 JP 2023003133
(71) Applicant: Yamaguchi University, Yamaguchi 753-8511 (JP)
(72) Inventor: MIZUNO, Takuya, Yamaguchi-shi, Yamaguchi 753-8515 (JP); NISHIBORI, Shoma, Yamaguchi-shi, Yamaguchi 753-8515 (JP)
(74) Representative: D Young & Co LLP
(86) International application number: PCT/JP2023/041176
(87) International publication number: WO 2024/150525

(57) **Abstract**

An object of the present invention is to provide, for example, an antibody that specifically binds to cat PD-1 and has action of inhibiting the binding between cat PD-1 and cat PD-L1. An antibody that specifically binds to cat PD-1 is obtained, the antibody comprising particular heavy chain complementarity-determining region (CDR) 1 to CDR3 and particular light chain CDR1 to CDR3.

## Description

### Technical Field

The present invention relates to: an antibody that specifically binds to cat PD-1 (programmed cell death 1), the antibody comprising particular heavy chain complementarity-determining region (CDR) 1 to CDR3 and particular light chain CDR1 to CDR3 (hereinafter, also referred to as the "present anti-cat PD1 antibody"); a polynucleotide encoding the present anti-cat PD1 antibody (hereinafter, also referred to as the "present anti-cat PD1 antibody-encoding polynucleotide"); a vector comprising the present anti-cat PD1 antibody-encoding polynucleotide operably linked downstream of a promoter (hereinafter, also referred to as the "present anti-cat PD1 antibody expression vector"); a host cell in which the present anti-cat PD1 antibody expression vector has been introduced (hereinafter, also referred to as the "present host cell"); an agent for inhibiting the binding between cat PD-1 and cat PD-L1, comprising the present anti-cat PD1 antibody (hereinafter, also referred to as the "present inhibitor"); a method for inhibiting the binding between cat PD-1 and cat PD-L1, comprising the step of administering the present anti-cat PD1 antibody to a cat (hereinafter, also referred to as the "present inhibition method"); a kit for inhibiting the binding between cat PD-1 and cat PD-L1, comprising the present anti-cat PD1 antibody or a labeled form thereof, or the present anti-cat PD1 antibody expression vector (hereinafter, also referred to as the "present kit for inhibition"); and the like.

### Background Art

Antibody drugs targeting immune checkpoint molecules which are immunosuppressive molecules have received attention for humans in recent years, and many clinical trials are underway. Such antibody drugs have produced excellent results, particularly, in clinical trials for human cancers targeting PD-1 (programmed cell death 1) and PD-L1 (programmed cell death 1 ligand-1), and have attracted interest as next-generation cancer treatment.

PD-1 is a receptor that resides on the surface of T cells. PD-1 has a function of suppressing the activation of T cells and has been found to have functions such as the suppression of immune response to self. Such suppression of immune response is performed by the binding of PD-L1, a PD-1 ligand, to PD-1. On the other hand, cancer cells express PD-L1 and acquire the ability to escape from immune response by suppressing the activation of T cells through the binding of the expressed PD-L1 to PD-1. Thus, the inhibition of the binding between PD-1 and PD-L1 is considered effective for the treatment of cancers.

A therapeutic agent for cancers, comprising an anti-PD-L1 antibody as an active ingredient and having action of suppressing the proliferation of cancer cells *in vivo* (patent document 1), and an anticancer agent comprising an anti-PD-1 antibody or an anti-PD-L1 antibody that restores the reactivity of iNKT cells with anergy induced by the administration of an iNKT cell ligand (patent document 2) have previously been proposed. Also, an anti-PD-1 antibody is under development as a therapeutic agent for melanoma, non-small cell lung cancer, and renal cell cancer (non-patent document 1).

Meanwhile, the number of cases that has rapidly increased with recent enhancement in the longevity of pets is cancers in pets. As in humans, advance has been achieved on cancer treatment for pets, and three major cancer therapies, i.e., surgery, radiotherapy, and chemotherapy (anticancer agent), are actively performed.

Among pets, dogs and cats have a high pet ownership rate. According to Japan Pet Food Association's 2020 national survey on dog and cat ownership, there are approximately 8.5 million pet dogs and approximately 9.6 million pet cats in Japan. The number of cancer cases developed in dogs or cats is also increasing with increase in the number of pet dogs or cats. The three major cancer therapies described above have treatment limitations, and novel treatment methods are thus demanded. Treatment targeting an immune checkpoint molecule such as PD-1 or PD-L1 is expected as such a method. Unfortunately, anti-human PD-1 antibodies or anti-human PD-L1 antibodies do not act on dogs or cats.

The present inventors have recently found an anti-canine PD-1 antibody or an anti-canine PD-L1 antibody that inhibits the binding between PD-1 and PD-L1 in dogs (patent document 3). On the other hand, for cats, research on PD-1 or PD-L1 has not yet proceeded, partly because researchers who conduct studies for cats are few in number. Thus, there is no previous report on an anti-cat PD-1 antibody or an anti-cat PD-L1 antibody.

### Prior Art Documents

### Patent Documents

Patent Document 1: Japanese unexamined Patent Application Publication No. 2014-65748
Patent Document 2: Japanese unexamined Patent Application Publication No. 2010-83863
Patent Document 3: International Publication No. WO 2016/006241

### Non-patent Documents

Non-patent Document 1: Suzanne L. et al., The New England Journal of Medicine Vol. 366, No. 26: 2443-2454, 2012

### Summary of the Invention

### Object to be Solved by the Invention

An object of the present invention is to provide, for example, an antibody that specifically binds to cat PD-1 and has action of inhibiting the binding between cat PD-1 and cat PD-L1.

### Means to Solve the Object

The present inventors are continuing diligent studies to solve the object. In the process, the present inventors have first confirmed that an anti-canine PD-1 antibody previously prepared by the present inventors (i.e., "3B7-D9" and "4F12-E6" in patent document 3) cannot detect a recombinant protein of cat PD-1. Accordingly, in order to prepare a mouse monoclonal antibody against cat PD-1, 288 clones were first obtained as a result of preparing a mouse cell line that expressed cat PD-1 (cat PD-1-expressing cell line), immunologically sensitizing mice with this cell line, then preparing fusion cells (hybridomas) of B cells (lymph node monocytes) and myeloma (P3U1 cells), and screening for cat PD-1-expressing cell line-positive hybridomas. From these 288 clones, four clones were obtained as a result of screening for hybridomas producing an antibody capable of binding to cat PD-1 in the cat PD-1-expressing cell line. From these four clones, two clones were obtained as a result of screening for hybridomas having action of inhibiting the binding between fPD1 and fPDL1-hIg. From these two clones, one clone was further obtained as a hybridoma producing an anti-fPD1 mouse monoclonal antibody having action of inhibiting the binding between fPD1 and fPDL1 as a result of single-cell cloning. The present invention has been completed on the basis of these findings.

Specifically, the present invention is as follows.
[1] An antibody that specifically binds to cat PD-1 consisting of the amino acid sequence represented by SEQ ID NO: 11, the antibody comprising
   heavy chain complementarity-determining region (CDR) 1 consisting of the amino acid sequence represented by SEQ ID NO: 1, heavy chain CDR2 consisting of the amino acid sequence represented by SEQ ID NO: 2, and heavy chain CDR3 consisting of the amino acid sequence represented by SEQ ID NO: 3, and
   light chain CDR1 consisting of the amino acid sequence represented by SEQ ID NO: 4, light chain CDR2 consisting of the amino acid sequence represented by SEQ ID NO: 5, and light chain CDR3 consisting of the amino acid sequence represented by SEQ ID NO: 6.
[2] The antibody according to [1] above, comprising a heavy chain variable region consisting of an amino acid sequence having 80% or higher sequence identity to the amino acid sequence represented by SEQ ID NO: 7, and a light chain variable region consisting of an amino acid sequence having 80% or higher sequence identity to the amino acid sequence represented by SEQ ID NO: 8.
[3] The antibody according to [1] or [2] above, comprising a heavy chain consisting of an amino acid sequence having 80% or higher sequence identity to the amino acid sequence represented by SEQ ID NO: 9, and a light chain consisting of an amino acid sequence having 80% or higher sequence identity to the amino acid sequence represented by SEQ ID NO: 10.
[4] A polynucleotide encoding the antibody according to any one of [1] to [3] above.
[5] A vector comprising a promoter and the polynucleotide according to [4] above operably linked downstream of the promoter.
[6] A host cell in which the vector according to [5] above has been introduced.
[7] An agent for inhibiting the binding between cat PD-1 and cat PD-L1, comprising the antibody according to any one of [1] to [3] above.
[8] A method for inhibiting the binding between cat PD-1 and cat PD-L1, comprising the step of administering the antibody according to any one of [1] to [3] above to a cat.
[9] A kit for inhibiting the binding between cat PD-1 and cat PD-L1, comprising the antibody according to any one of [1] to [3] above or a labeled form thereof, or the vector according to [5] above.

Other examples of the mode of carrying out the invention can include the present anti-cat PD1 antibody for use in immunotherapy (e.g., cancer immunotherapy) for a cat, and use of the present anti-cat PD1 antibody for producing a formulation for immunotherapy (e.g., cancer immunotherapy) for a cat.

### Effect of the Invention

According to the present invention, an antibody that specifically binds to cat PD-1 and has action of inhibiting the binding between cat PD-1 and cat PD-L1 can cancel the suppression of T cell activation by the binding between fPD1 and fPDL1 in a cat and activate T cells, and is therefore useful in immunotherapy (e.g., cancer immunotherapy) for cats.

### Brief Description of Drawings

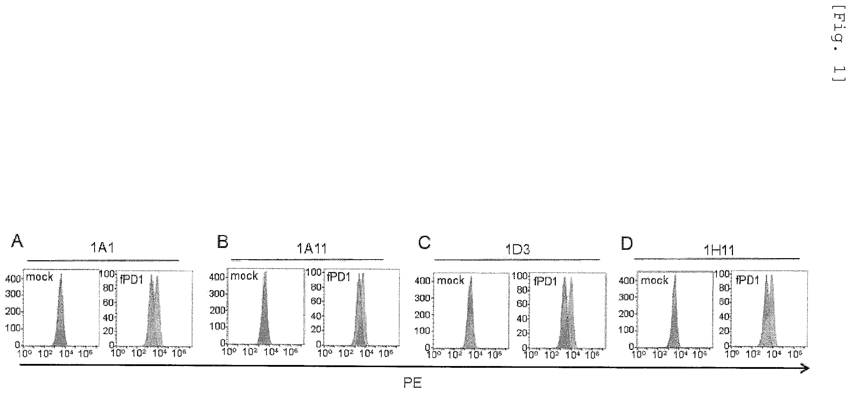

[Figure 1] Figure 1 is a diagram showing results of incubating an NIH3T3 cell line stably expressing cat (*Felis silvestris catus*) PD-1 (hereinafter, also referred to as "fPD1") (hereinafter, this cell line is also referred to as "NIH3T3/fPD1") ("fPD1" in the drawing) or an NIH3T3 cell line (hereinafter, also referred to as "NIH3T3") ("mock" in the drawing) in the presence of four types of anti-fPD1 mouse monoclonal antibodies (1A1 [Figure 1A], 1A11 [Figure 1B], 1D3 [Figure 1C], and 1H11 [Figure 1D]), and incubating the cell line in the presence of anti-mouse IgG-PE, followed by flow cytometry analysis. "PE" of the abscissa depicts PE-derived fluorescence intensity in anti-mouse IgG-PE, and the ordinate depicts the number of cells. The direction of the arrow of the abscissa represents higher fluorescent brightness (the same holds true for the description below).

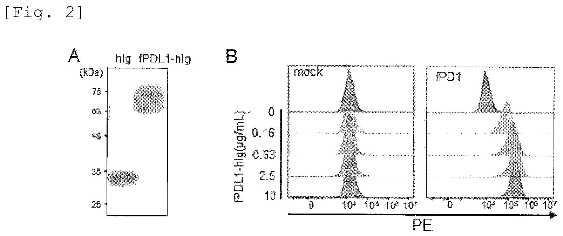

[Figure 2] Figure 2A is a diagram showing results of detecting a fusion protein of a cat PD-L1 (hereinafter, also referred to as "fPDL1") extracellular region and a human IgG2 Fc region (hereinafter, this fusion protein is also referred to as "fPDL1-hIg"), and a human IgG2 Fc region (hereinafter, also referred to as "hIg") by Western blotting using anti-human IgG. Figure 2B is a diagram showing results of incubating NIH3T3/fPD1 ("fPD1" in the drawing) or NIH3T3 ("mock" in the drawing) in the presence of fPDL1-hIg, and incubating the cell line in the presence of anti-human IgG-PE, followed by flow cytometry analysis. "PE" of the abscissa depicts PE-derived fluorescence intensity in anti-human IgG-PE, and the ordinate depicts the number of cells.

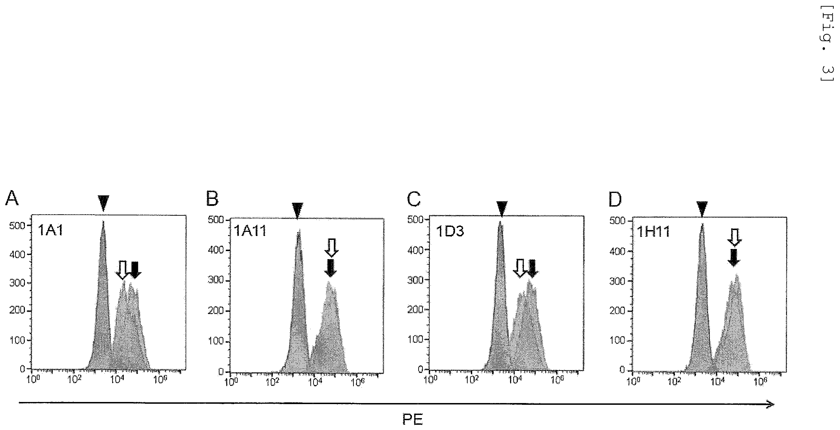

[Figure 3] Figure 3 is a diagram showing results of 1) incubating NIH3T3/fPD1 in the presence of four types of anti-fPD1 mouse monoclonal antibodies (1A1 [Figure 3A], 1A11 [Figure 3B], 1D3 [Figure 3C], and 1H11 [Figure 3D]) (arrowhead in the drawing), 2) incubating the cell line in the presence of fPDL1-hIg (arrow in the drawing), or 3) incubating the cell line in the presence of the four types of anti-fPD1 mouse monoclonal antibodies and fPDL1-hIg (open arrow in the drawing), and incubating the cell line in the presence of anti-human IgG-PE, followed by flow cytometry analysis. "PE" of the abscissa depicts PE-derived fluorescence intensity in anti-human IgG-PE, and the ordinate depicts the number of cells.

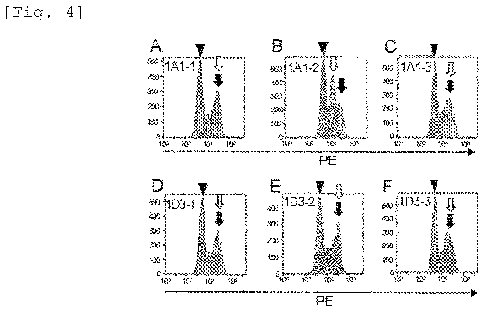

[Figure 4] Figure 4 is a diagram showing results of 1) incubating NIH3T3/fPD1 in the presence of six types of anti-fPD1 mouse monoclonal antibodies (1A1-1 [Figure 4A], 1A1-2 [Figure 4B], 1A1-3 [Figure 4C], 1D3-1 [Figure 4D], 1D3-2 [Figure 4E], and 1D3-3 [Figure 4F]) (arrowhead in the drawing), 2) incubating the cell line in the presence of fPDL1-hIg (arrow in the drawing), or 3) incubating the cell line in the presence of the six types of anti-fPD1 mouse monoclonal antibodies and fPDL1-hIg (open arrow in the drawing), and incubating the cell line in the presence of anti-human IgG-PE, followed by flow cytometry analysis. "PE" of the abscissa depicts PE-derived fluorescence intensity in anti-human IgG-PE, and the ordinate depicts the number of cells.

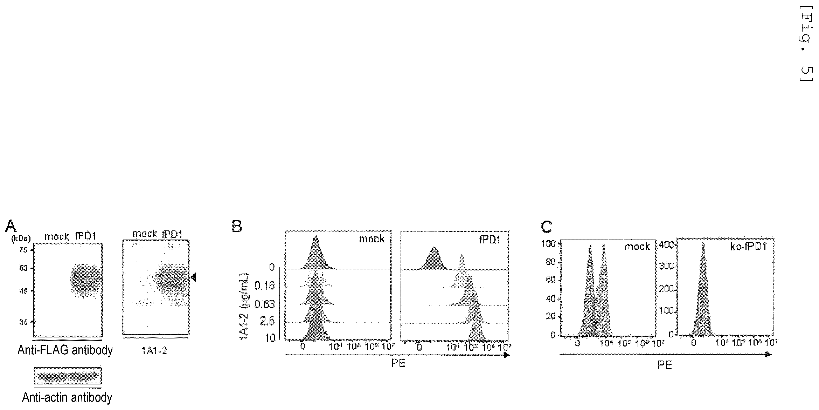

[Figure 5] Figure 5A is a diagram showing results of detecting NIH3T3/fPD1 by Western blotting using an anti-FLAG antibody (let box) and "1A1-2" (right box). Figure 5B is a diagram showing results of incubating NIH3T3/fPD1 ("fPD1" in the drawing) or NIH3T3 ("mock" in the drawing) in the presence of "1A1-2", and incubating the cell line in the presence of anti-mouse IgG-PE, followed by flow cytometry analysis. Figure 5C is a diagram showing results of incubating fPD1 gene-knockout cat lymphoma cell line (FT-1) (hereinafter, also referred to as "FT-1/ko-fPD1") or FT-1 in the presence of "1A1-2", and incubating the cell line in the presence of anti-mouse IgG-PE, followed by flow cytometry analysis. In Figures 5B and 5C, "PE" of the abscissa depicts PE-derived fluorescence intensity in anti-mouse IgG-PE, and the ordinate depicts the number of cells.

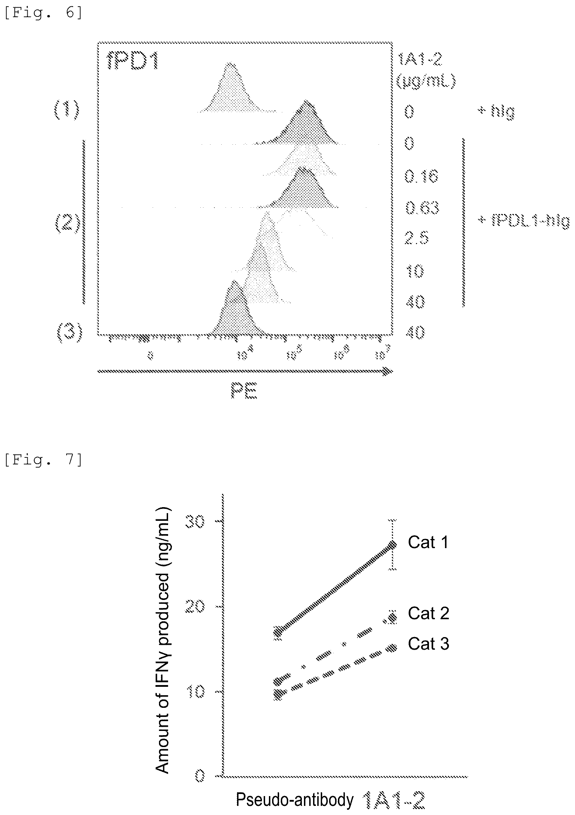

[Figure 6] Figure 6 is a diagram showing results of 1) incubating NIH3T3/fPD1 in the absence of "1A1-2" and in the presence of hIg ((1) in the drawing), 2) incubating the cell line in the presence or absence of "1A1-2" and in the presence of fPDL1-hIg ((2) in the drawing), or 3) incubating the cell line in the presence of "1A1-2" ((3) in the drawing), and incubating the cell line in the presence of anti-human IgG-PE, followed by flow cytometry analysis. "PE" of the abscissa depicts PE-derived fluorescence intensity in anti-human IgG-PE, and the ordinate depicts the number of cells.
[Figure 7] Figure 7 is a diagram showing results of culturing peripheral blood mononuclear cells (PBMC) derived from three healthy cats (cat 1, cat 2, and cat 3) in the presence of concanavalin A (ConA) and an isotype control (pseudo-antibody) or "1A1-2", and measuring the concentration of cat IFN-γ (interferon-γ; hereinafter, also referred to as "fIFNγ") in each culture solution. A statistically significant difference (p = 0.03091596) was found between the pseudo-antibody and "1A1-2" by the paired t test.

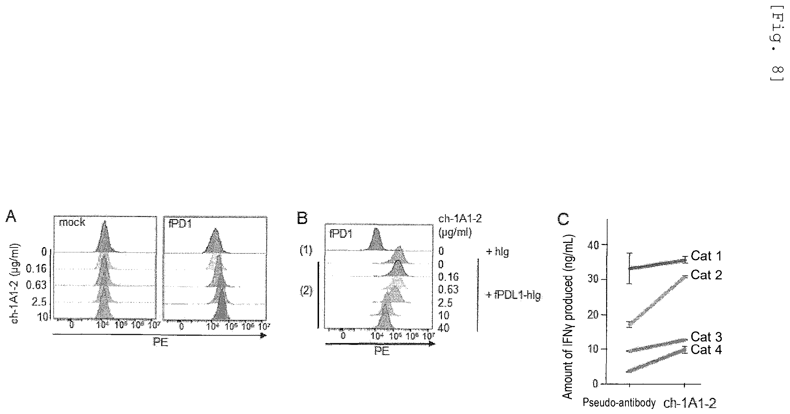

[Figure 8] Figure 8A is a diagram showing results of incubating NIH3T3/fPD1 ("fPD1" in the drawing) or NIH3T3 ("mock" in the drawing) in the presence of "ch-1A1-2" (i.e., a chimeric antibody consisting of a heavy chain variable region of "1A1-2" and a constant region of cat IgG1, and a light chain variable region of "1A1-2" and a constant region of cat IgG1), and incubating the cell line in the presence of anti-cat IgG-PE, followed by flow cytometry analysis. "PE" of the abscissa depicts PE-derived fluorescence intensity in anti-cat IgG-PE, and the ordinate depicts the number of cells. Figure 8B is a diagram showing results of 1) incubating NIH3T3/fPD1 in the absence of "ch-1A1-2" and in the presence of hIg ((1) in the drawing), or 2) incubating the cell line in the presence or absence of "ch-1A1-2" and in the presence of fPDL1-hIg ((2)in the drawing), and incubating the cell line in the presence of anti-human IgG-PE, followed by flow cytometry analysis. "PE" of the abscissa depicts PE-derived fluorescence intensity in anti-human IgG-PE, and the ordinate depicts the number of cells.
Figure 8C is a diagram showing results of culturing PBMC derived from four healthy cats (cat 1, cat 2, cat 3, and cat 4) in the presence of ConA and an isotype control (pseudo-antibody) or "ch-1A1-2", and measuring the concentration of fIFNγ in each culture solution.

### Mode of Carrying Out the Invention

### <1. Present anti-cat PD1 antibody>

The antibody of the present invention is an antibody that specifically binds to cat PD-1 consisting of the amino acid sequence of SEQ ID NO: 11 (i.e., an antibody that recognizes and binds to a polypeptide consisting of the amino acid sequence of SEQ ID NO: 11 through a highly specific antigen-antibody recognition mechanism), the antibody comprising heavy (H) chain CDR1 consisting of the amino acid sequence of SEQ ID NO: 1, H chain CDR2 consisting of the amino acid sequence of SEQ ID NO: 2, and H chain CDR3 consisting of the amino acid sequence of SEQ ID NO: 3, and light (L) chain CDR1 consisting of the amino acid sequence of SEQ ID NO: 4, L chain CDR2 consisting of the amino acid sequence of SEQ ID NO: 5, and L chain CDR3 consisting of the amino acid sequence of SEQ ID NO: 6 (i.e., the present anti-cat PD1 antibody). The antibody of the present invention has action of inhibiting the binding between cat PD-1 and cat PD-L1. Such action can cancel the suppression of cat T cell activation by the binding between cat PD1 and cat PDL1 and activate cat T cells. The H chain CDR1 to CDR3 are usually contained in an H chain variable region, and the L chain CDR1 to CDR3 are usually contained in an L chain variable region.

In the present specification, the "cat" means any of a domestic cat (*Felis silvestris catus*), a wildcat, and an alley cat (feral cat) which are small mammals classified into order *Carnivora-suborder Feliformia*-family *Felidae-*subfamily Felinae-genus *Felis.* An animal of the family *Felidae* (e.g., lion, tiger, and leopard) other than the cat is not included in the "cat".

The present anti-cat PD1 antibody is not particularly limited by its origin, type, class, form, or the like and includes: a mouse-, rat-, or cat-derived antibody; a polyclonal antibody, an oligoclonal antibody (mixture of several to several tens of antibodies), and a monoclonal antibody; and a chimeric antibody in which a partial region (e.g., constant regions) of an antibody has been substituted by a region derived from a different organism species (e.g., a cat), an antibody fragment such as a F(ab')₂ antibody fragment obtained by digesting a monoclonal antibody with pepsin, a Fab' antibody fragment obtained by reducing a F(ab')₂ antibody fragment, and Fab obtained by digesting a monoclonal antibody with papain, and a recombinant antibody such as scFv containing an antibody heavy (H) chain variable region and an antibody light (H) chain variable region linked through amino acid cross-link. The present anti-cat PD1 antibody is preferably a monoclonal antibody, more preferably a monoclonal antibody comprising cat-derived constant regions.

The present anti-cat PD1 antibody preferably in a separated form. In this context, the term "separated" means that the antibody is present in a state different from the state where the antibody is originally present in such a way that the antibody is taken out of an environment originally involving the antibody or expressed in an environment different from the environment originally involving the antibody by an artificial operation. Specifically, the "separated antibody" does not include an antibody that is derived from a certain individual and is in a state contained in the body of the individual without an external operation (artificial operation) or in a tissue or a body fluid (blood, plasma, serum, etc.) derived from the body. The present anti-cat PD1 antibody is preferably an antibody prepared by an artificial operation (e.g., the recombinant antibody described above). Such an "antibody derived from a cell prepared by an artificial operation or an antibody produced from the cell" does not include an antibody that is not subjected to an artificial operation, for example, an antibody produced from a naturally occurring B cell.

In the present anti-cat PD1 antibody, a framework region (FR) is usually linked to the amino (N) terminus and/or carboxyl (C) terminus of each of H chain CDR1 to CDR3 and L chain CDR1 to CDR3. Among such FRs, examples of the H chain FRs can include H chain FR1 linked to the N terminus of H chain CDR1, H chain FR2 linked to the C terminus of H chain CDR1 (N terminus of H chain CDR2), H chain FR3 linked to the C terminus of H chain CDR2 (N terminus of H chain CDR3), and H chain FR4 linked to the C terminus of H chain CDR3. Among the FRs, examples of the L chain FRs can include L chain FR1 linked to the N terminus of L chain CDR1, L chain FR2 linked to the C terminus of L chain CDR1 (N terminus of L chain CDR2), L chain FR3 linked to the C terminus of L chain CDR2 (N terminus of L chain CDR3), and L chain FR4 linked to the C terminus of L chain CDR3.

Examples of the H chain FR1 can include a polypeptide consisting of amino acid residues 1 to 30 in the amino acid sequence of SEQ ID NO: 7, and a polypeptide consisting of an amino acid sequence having 80% or higher sequence identity to the amino acid sequence of the polypeptide.

Examples of the H chain FR2 can include a polypeptide consisting of amino acid residues 36 to 49 in the amino acid sequence of SEQ ID NO: 7, and a polypeptide consisting of an amino acid sequence having 80% or higher sequence identity to the amino acid sequence of the polypeptide.

Examples of the H chain FR3 can include a polypeptide consisting of amino acid residues 67 to 98 in the amino acid sequence of SEQ ID NO: 7, and a polypeptide consisting of an amino acid sequence having 80% or higher sequence identity to the amino acid sequence of the polypeptide.

Examples of the H chain FR4 can include a polypeptide consisting of amino acid residues 114 to 124 in the amino acid sequence of SEQ ID NO: 7, and a polypeptide consisting of an amino acid sequence having 80% or higher sequence identity to the amino acid sequence of the polypeptide.

Examples of the L chain FR1 can include a polypeptide consisting of amino acid residues 1 to 23 in the amino acid sequence of SEQ ID NO: 8, and a polypeptide consisting of an amino acid sequence having 80% or higher sequence identity to the amino acid sequence of the polypeptide.

Examples of the L chain FR2 can include a polypeptide consisting of amino acid residues 35 to 49 in the amino acid sequence of SEQ ID NO: 8, and a polypeptide consisting of an amino acid sequence having 80% or higher sequence identity to the amino acid sequence of the polypeptide.

Examples of the L chain FR3 can include a polypeptide consisting of amino acid residues 57 to 88 in the amino acid sequence of SEQ ID NO: 8, and a polypeptide consisting of an amino acid sequence having 80% or higher sequence identity to the amino acid sequence of the polypeptide.

Examples of the L chain FR4 can include a polypeptide consisting of amino acid residues 98 to 109 in the amino acid sequence of SEQ ID NO: 8, and a polypeptide consisting of an amino acid sequence having 80% or higher sequence identity to the amino acid sequence of the polypeptide.

The H chain CDR1 in the present anti-cat PD1 antibody usually resides at positions H31 to H35 based on the Kabat numbering (see the document "Kabat, E. A. et al., (1991) NIH Publication No. 91-3242, sequences of proteins of immunological interest"). The H chain CDR2 in the present anti-cat PD1 antibody usually resides at positions H50 to H52, H52A, and H53 to H65 based on the Kabat numbering. The H chain CDR3 in the present anti-cat PD1 antibody usually resides at positions H95 to H100, H100A to H100G, H101, and H102 based on the Kabat numbering. The L chain CDR1 in the present anti-cat PD1 antibody usually resides at positions L24 to L34 based on the Kabat numbering. The L chain CDR2 in the present anti-cat PD1 antibody usually resides at positions L50 to L56 based on the Kabat numbering. The L chain CDR3 in the present anti-cat PD1 antibody usually resides at positions L89 to L97 based on the Kabat numbering.

The present anti-cat PD1 antibody preferably comprises an H chain variable region consisting of an amino acid sequence having 80% or higher sequence identity to the amino acid sequence of SEQ ID NO: 7, and an L chain variable region consisting of an amino acid sequence having 80% or higher sequence identity to the amino acid sequence of SEQ ID NO: 8, and more preferably comprises an H chain consisting of an amino acid sequence having 80% or higher sequence identity to the amino acid sequence of SEQ ID NO: 9, and an L chain consisting of an amino acid sequence having 80% or higher sequence identity to the amino acid sequence of SEQ ID NO: 10.

The present anti-cat PD1 antibody can be obtained by transfecting a host cell with the present anti-cat PD1 antibody expression vector, culturing the prepared cell (i.e., the present host cell) in a culture solution appropriate for the host cell, and recovering the present anti-cat PD1 antibody produced in the culture solution.

A transgenic animal, such as a mouse, a bovine, a goat, sheep, a chicken, or a pig, in which a portion or the whole of the present anti-cat PD1 antibody expression vector has been incorporated is prepared by use of a transgenic animal preparation technique, and the present anti-cat PD1 antibody can also be produced in a large amount from the blood, milk, or the like of the transgenic animal.

A nonhuman animal (e.g., a mouse or a rat) is immunized with a nonhuman animal (e.g., mouse or rat) cell line expressing cat PD-1 consisting of the amino acid sequence of SEQ ID NO: 11 (cat PD-1-expressing nonhuman animal cell line). Antibody-producing hybridomas are prepared by use of a cell fusion technique. A hybridoma producing the present anti-cat PD1 antibody can be obtained by 1) screening for an anti-cat PD-1 antibody-producing hybridoma by ELISA using a cat PD-1-immobilized plate and/or 2) screening for a hybridoma positive for the cat PD-1-expressing nonhuman animal cell line by flow cytometry. Polynucleotides encoding constant regions in the present anti-cat PD1 antibody-encoding polynucleotide contained in the genomic DNA of the hybridoma may be substituted by polynucleotides encoding constant regions derived from a different organism species (e.g., a cat) through the use of CRISPR/Cas9 system in accordance with a method described in the document "Sci Adv. 2019 Aug 28; 5 (8): eaaw1822" or the document "Vet Comp Oncol. 2020 Dec; 18 (4): 739-752" to prepare a hybridoma producing a chimeric antibody having variable regions and constant regions derived from different organism species. The present anti-cat PD1 antibody can be purified from a hybridoma culture supernatant by use of an antibody purification technique known in the art.

### <2. Present anti-cat PD1 antibody-encoding polynucleotide>

The polynucleotide of the present invention can be a polynucleotide encoding the present anti-cat PD1 antibody (i.e., the present anti-cat PD1 antibody-encoding polynucleotide). A nucleotide constituting the present anti-cat PD1 antibody-encoding polynucleotide may be DNA or RNA having a natural structure or may be a nucleotide having a chemically modified structure of DNA or RNA having a natural structure (also referred to as a "modified nucleic acid"). When the present anti-cat PD1 antibody-encoding polynucleotide is, for example, in the form of mRNA, a modified nucleic acid is used in order to confer resistance to degradation by RNase. The modified nucleic acid preferably has a modified base moiety in the nucleotide and is preferably, for example, 5-substituted pyrimidine nucleotide or optionally 1-substituted pseudouridine. Specific examples thereof can include 5-methylcytidine, 5-methoxyuridine, 5-methyluridine, pseudouridine, and 1-alkylpseudouridine. The 1-alkylpseudouridine can be 1-(C1-C6 alkyl)pseudouridine and is preferably 1-methylpseudouridine or 1-ethylpseudouridine.

The present anti-cat PD1 antibody-encoding polynucleotide can be easily prepared by a routine method on the basis of the amino acid sequence of the present anti-cat PD1 antibody. A nucleotide sequence encoding the amino acid sequence can be obtained on the basis of an amino acid sequence described in the sequence listing, and the present anti-cat PD1 antibody-encoding polynucleotide can be prepared by use of standard molecular biological and/or chemical procedures.

The present anti-cat PD1 antibody-encoding polynucleotide may comprise a nucleotide sequence codon-optimized for expression in a particular host cell. The nucleotide sequence thus optimized can be obtained by applying an algorithm or software known in the art to the target amino acid sequence.

The present anti-cat PD1 antibody-encoding polynucleotide may be a single-stranded (sense strand) polynucleotide encoding the present anti-cat PD1 antibody or may be a double-stranded polynucleotide consisting of the sense strand and an antisense strand having a sequence complementary thereto. The form thereof is selected as a form suitable for a method for introducing the polynucleotide to cells. For example, mRNA or a lentivirus vector is in a single-stranded form, and plasmid DNA is capable of assuming a double-stranded form.

The present anti-cat PD1 antibody-encoding polynucleotide preferably comprises a polynucleotide encoding an H chain variable region consisting of an amino acid sequence having 80% or higher sequence identity to the amino acid sequence of SEQ ID NO: 7, and a polynucleotide encoding an L chain variable region consisting of an amino acid sequence having 80% or higher sequence identity to the amino acid sequence of SEQ ID NO: 8, and more preferably comprises a polynucleotide encoding an H chain consisting of an amino acid sequence having 80% or higher sequence identity to the amino acid sequence of SEQ ID NO: 9, and a polynucleotide encoding an L chain consisting of an amino acid sequence having 80% or higher sequence identity to the amino acid sequence of SEQ ID NO: 10.

In the present specification, the "80% or higher identity" means that the identity is 80% or higher (at least 80%), preferably 85% or higher, more preferably 88% or higher, further preferably 90% or higher, still further preferably 93% or higher, particularly preferably 95% or higher, particularly more preferably 98% or higher, most preferably 100%.

In the present specification, the term "identity" means the degree of similarity between polypeptide or polynucleotide sequences (this degree is determined by matching a query sequence to another sequence, preferably of the same type (nucleic acid or protein sequence). Examples of a preferred computer program method for calculating and determining the "identity" include, but are not limited to, GCG BLAST (Basic Local Alignment Search Tool) (Altschul et al., J. Mol. Biol. 1990, 215: 403-410; Altschul et al., Nucleic Acids Res. 1997, 25: 3389-3402; and Devereux et al., Nucleic Acid Res. 1984,12: 387), BLASTN 2.0 (Gish W., http://blast.wustl.edu,1996-2002), FASTA (Pearson and Lipman, Proc. Natl. Acad. Sci. USA 1988, 85: 2444-2448), and GCG GelMerge which determines and aligns a pair of the longest overlapping contigs (Wibur and Lipman, SIAM J. Appl. Math. 1984, 44: 557-567; and Needleman and Wunsch, J. Mol. Biol. 1970, 48: 443-453).

In the present specification, the "amino acid sequence having 80% or higher sequence identity to the amino acid sequence represented by SEQ ID NO: X (amino acid sequence of SEQ ID NO: X)" is, in other words, an "amino acid sequence that is derived from the amino acid sequence represented by SEQ ID NO: X (amino acid sequence of SEQ ID NO: X) by the deletion, substitution, insertion, and/or addition of 0, 1 or several amino acid residues and has functions equivalent to those of the amino acid sequence represented by SEQ ID NO: X (amino acid sequence of SEQ ID NO: X)". In this context, the "amino acid sequence derived by the deletion, substitution, insertion, and/or addition of 1 or several amino acid residues" means an amino acid sequence in which amino acid residues have been deleted, substituted, inserted, and/or added, for example, within the range of 1 to 30 residues, preferably within the range of 1 to 20 residues, more preferably within the range of 1 to 15 residues, further preferably within the range of 1 to 10 residues, further preferably within the range of 1 to 5 residues, further preferably within the range of 1 to 3 residues, further preferably within the range of 1 or 2 residues. The mutation treatment of these amino acid residues can be performed by an arbitrary method known to those skilled in the art such as chemical synthesis, a gene engineering approach, or mutagenesis.

### <3. Present anti-cat PD1 antibody expression vector>

The vector of the present invention is not particularly limited as long as the vector comprises a promoter and the present anti-cat PD1 antibody-encoding polynucleotide operably linked downstream of the promoter and can transcribe mRNA encoded by the present anti-cat PD1 antibody-encoding polynucleotide (i.e., the present anti-cat PD1 antibody expression vector).

The present anti-cat PD1 antibody expression vector can be appropriately selected depending on a purpose. Examples thereof can include a nonviral vector (e.g., an episomal vector, an artificial chromosome vector, and a plasmid vector) and a virus vector. The vector may be circular or linear.

The promoter in the present anti-cat PD1 antibody expression vector is not particularly limited as long as the region starts the transcription of mRNA encoded by the present polynucleotide located downstream of the promoter through the binding of RNA polymerase (preferably RNA polymerase and a general transcription factor). Examples thereof can include SRα promoter, SV40 early promoter, viral LTR (long terminal repeat), CMV (cytomegalovirus) promoter, RSV (Rous sarcoma virus) promoter, HSV-TK (helps simplex virus thymidine kinase) promoter, EF1α promoter, metallothionein promoter, and heat shock promoter.

The present anti-cat PD1 antibody expression vector may contain, if desired, an enhancer, a poly-A addition signal, a marker gene, a replication origin, a gene encoding a polypeptide that controls replication by binding to the replication origin, or the like, in addition to the promoter. Such a marker gene refers to a gene that enables a cell to be screened for or selected by the transfection of the cell with the marker gene. Specific examples of the marker gene can include a drug resistance gene, a fluorescence protein gene, a luminescence enzyme gene, and a chromogenic enzyme gene. One of these marker genes may be used singly, or two or more thereof may be used in combination. Specific examples of the drug resistance gene can include neomycin resistance gene, tetracycline resistance gene, kanamycin resistance gene, zeocin resistance gene, and hygromycin resistance gene. Specific examples of the fluorescence protein gene can include blue fluorescence protein (BFP), green fluorescence protein (GFP) gene, yellow fluorescence protein (YFP) gene, and red fluorescence protein (RFP) gene. Specific examples of the luminescence enzyme gene can include luciferase gene. Specific examples of the chromogenic enzyme gene can include β galactosidase gene, β glucuronidase gene, and alkaline phosphatase gene.

### <4. Present host cell>

The host cell of the present invention is not particularly limited as long as it is a cell in which the present anti-cat PD1 antibody expression vector has been introduced and expresses the present anti-cat PD1 antibody (i.e., the present host cell). The present host cell usually survives or is maintained in a liquid or in a state moistened (wetted) with a liquid in a container (e.g., a culture plate or dish, or a tube for cell preservation or cell sorting). Such a liquid is not particularly limited as long as the present host cell can survive or be maintained therein. Examples of the liquid can include a culture solution (e.g., a serum-containing or serum-free culture solution), saline, phosphate-buffered saline, Tris-buffered saline, HEPES-buffered saline, Ringer's solution (lactate Ringer's solution, acetate Ringer's solution, bicarbonate Ringer's solution, etc.), and a 5% aqueous glucose solution. Examples of the serum can include 0.1 to 30 (v/v) % serum (fetal bovine serum [FBS], calf bovine serum [CS], etc.). Examples of the culture solution can include a culture solution for animal cell culture (DMEM, EMEM, IMDM, RPMI1640, αMEM, F-12, F-10, M-199, AIM-V, etc.). Examples of the serum-free culture solution (serumless culture solution) can include the culture solution for animal cell culture supplemented with a serum replacement such as commercially available B27 supplement (-insulin) (manufactured by Life Technologies Corp.), N2 supplement (manufactured by Life Technologies Corp.), B27 supplement (manufactured by Life Technologies Corp.), or Knockout Serum Replacement (manufactured by Invitrogen Corp.) in an appropriate amount (e.g., 1 to 30%).

The present host cell can be prepared by transfecting a host cell with the present anti-cat PD1 antibody expression vector. A method for transfecting a host cell with the present anti-cat PD1 antibody expression vector can be a method suitable for the present anti-cat PD1 antibody expression vector and the host cell. In the case of using, for example, a nonviral vector, as the present anti-cat PD1 antibody expression vector, examples thereof can include: a method using a fine particle such as a liposome or a cationic lipid as described in International Publication Nos. Wo 96/10038, WO 97/18185, WO 97/25329, WO 97/30170, and WO 97/31934 (which are incorporated herein by reference in their entirety); a method of introducing an episomal vector for scaffold/matrix attachment region element expression described in the document "Jin et al, EMBO Mol Med. 2016 Jul; 8 (7): 702-711"; a method using an outer shell of a virus particle, the contents of which have been destroyed by radiation, which is adopted to a transposon-based introduction method such as PiggyBac method described in the document "Mol Ther Methods Clin Dev. 2017 Dec 22;8:131-140"; and a method of introducing the present anti-cat PD1 antibody expression vector (specifically, the present anti-cat PD1 antibody expression vector in a linear form) to a target cell genome by a genome editing technique using, for example, CRISPR/Cas9 system or Zn finger nuclease (e.g., U.S. Patent No. 8,956,828).

In the case of using a virus vector (i.e., a recombinant virus) as the present anti-cat PD1 antibody expression vector, examples of the method for transfecting a host cell with the present anti-cat PD1 antibody expression vector can include a method of virally infecting an immunocyte using a culture supernatant containing a recombinant virus (i.e., a virus particle containing a virus vector plasmid [which may be DNA or RNA] in which a foreign gene [in the case of the present application, the present anti-cat PD1 antibody-encoding polynucleotide] has been incorporated in place of a pathogenicity-related gene removed from the virus genome), or an enriched recombinant virus.

The host cell can be any cell that expresses the present anti-cat PD1 antibody by the transcription of the present anti-cat PD1 antibody-encoding polynucleotide. Examples thereof can include a yeast, a mammalian (e.g., human, mouse, rat, canine, or cat) cell, an insect (e.g., *Spodoptera frugiperda* or *Trichoplusiani*) cell, and a plant (e.g., tobacco, potato, tomato, carrot, soybean, turnip rape, alfalfa, rice, wheat, or barley) cell.

### <5. Present agent for inhibiting the binding>

The agent for inhibiting the binding between cat PD-1 and cat PD-L1 of the present invention is an agent specified for the purpose of "inhibiting the binding between cat PD-1 and cat PD-L1", the agent containing the present anti-cat PD1 antibody (i.e., the present inhibitor). The "binding between cat PD-1 and cat PD-L1" to be inhibited may be within a cat culture cell or may be within the cat body.

For the present inhibitor, the present anti-cat PD1 antibody serving as an active ingredient may be used alone as a food or drink product for cats or a medicament (formulation) for cats or may be further mixed with an additive and used in the form of a composition (food or drink composition for cats or pharmaceutical composition for cats).

Examples of the additive for the present inhibitor can include an ingredient such as a physiologically acceptable usual carrier, a binder, a stabilizer, an excipient, a diluent, a pH buffering agent, a disintegrant, a tonicity agent, a coating agent, a solubilizer, a lubricating agent, a sliding agent, a dissolution aid, a lubricant, a flavor, a sweetener, a solvent, a gelling agent, and a nutrient. Specific examples of such an ingredient can include water, saline, animal fat and oil, plant oil, lactose, starch, gelatin, crystalline cellulose, gum, talc, magnesium stearate, hydroxypropylcellulose, polyalkylene glycol, polyvinyl alcohol, and glycerin.

The present inhibitor may comprise a component that inhibits the binding between cat PD-1 and cat PD-L1, in addition to the present anti-cat PD1 antibody serving as an active ingredient, and preferably comprises no component (e.g., compound, protein [e.g., antibody], DNA, or RNA) that inhibits the binding between cat PD-1 and cat PD-L1, in addition to the present anti-cat PD1 antibody because the present anti-cat PD1 antibody alone exerts an excellent inhibitory effect.

The present inhibitor has action of inhibiting the binding between cat PD-1 and cat PD-L1, cancelling the suppression of T cell activation in a cat, and activating T cells in the cat, and can therefore be advantageously applied to a formulation for immunotherapy (e.g., cancer immunotherapy) for a cat.

### <6. Present inhibition method>

The method for inhibiting the binding between cat PD-1 and cat PD-L1 according to the present invention is not particularly limited as long as the method comprises the step of administering the present anti-cat PD1 antibody to a cat and inhibits the binding between PD-1 and PD-L1 in the cat body (i.e., the present inhibition method).

The recipient cat for the administration of the present anti-cat PD1 antibody can be any cat in need of inhibiting the binding between cat PD-1 and cat PD-L1. Specific examples thereof can include a cat in need of cancelling the suppression of T cell activation by the binding between PD1 and PDL1 and activating T cells. More specific examples thereof can include a cat in need of immunotherapy (e.g., cancer immunotherapy) (e.g., a cat with a cancer and a cat having the risk of having a cancer).

Examples of the administration mode of the present anti-cat PD1 antibody can include oral administration that is performed in a dosage form such as a powder, a granule, a tablet, a capsule, or a syrup, and parenteral administration that is performed in a dosage form such as a skin external preparation, a transdermal agent, a transmucosal agent, a transnasal agent, an enteral agent, an injection, a suppository, an inhalant, or a patch.

The dose of the present anti-cat PD1 antibody is appropriately determined depending on the age, body weight, sex, symptom, sensitivity to a drug, or the like of the cat and is in a dose range of, for example, 1 µg to 100 mg/kg (body weight) /day. The present anti-cat PD1 antibody is administered in a single dose or multiple doses (e.g., 2 to 4 doses) per day, and the dose may be adjusted depending on the status of improvement in the symptom of the cat.

The present inhibition method can administer the present anti-cat PD1 antibody to a cat, thereby inhibiting the binding between cat PD-1 and cat PD-L1, cancelling the suppression of T cell activation in the cat, and activating T cells in the cat, and can therefore be advantageously applied to immunotherapy (e.g., cancer immunotherapy) for a cat.

### <7. Present kit for inhibition>

The kit for inhibiting the binding between cat PD-1 and cat PD-L1 according to the present invention is a kit specified for the purpose of "inhibiting the binding between cat PD-1 and cat PD-L1", the kit comprising the present anti-cat PD1 antibody or a labeled form thereof, or the present anti-cat PD1 antibody expression vector (i.e., the present kit for inhibition). The kit usually comprises a component generally used in this kind of kit, for example, a carrier, a pH buffering agent, a stabilizer, a manual, and an instruction for inhibiting the binding between cat PD-1 and cat PD-L1. The "binding between cat PD-1 and cat PD-L1" to be inhibited may be within a cat culture cell or may be within the cat body.

Examples of the labeling material for the labeled form of the present anti-cat PD1 antibody can include: an enzyme such as peroxidase (e.g., horseradish peroxidase [HRP]), alkaline phosphatase, β-D-galactosidase, glucose oxidase, glucose-6-phosphate dehydrogenase, alcohol dehydrogenase, malate dehydrogenase, penicillinase, catalase, apo-glucose oxidase, urease, luciferase, and acetylcholinesterase; a fluorescent material such as fluorescein isothiocyanate, phycobiliprotein, rare earth metal chelate, dansyl chloride, and tetramethylrhodamine isothiocyanate; a fluorescence protein such as green fluorescence protein (GFP), cyan fluorescence protein (CFP), blue fluorescence protein (BFP), yellow fluorescence protein (YFP), red fluorescence protein (RFP), and luciferase; a radioisotope such as ³H, ¹⁴C, ¹²⁵I, and ¹³¹I; biotin; avidin; and a chemiluminescence material.

Hereinafter, the present invention will be more specifically described with reference to Examples. However, the technical scope of the present invention is not limited by these examples. In Examples given below, primers used in PCR are shown in Table 1. An NIH3T3 cell line, a mouse fibroblast line, used in Examples given below was cultured and maintained under conditions of 5% CO₂ and 37°C in DMEM culture solution containing 10% FBS, 100 units/ml of penicillin, 100 µg/ml of streptomycin, and 55 µM 2-mercaptoethanol (hereinafter, simply referred to as "DMEM culture solution").

**[Table 1]**

| Primer name | Nucleotide sequence | SEQ ID NO |
|---|---|---|
| Forward primer 1 | GTGGATCCCGTGGAGGAAGAGATTACGG (recognition site for restriction enzyme BamHI is underlined) | 13 |
| Reverse primer 1 | TCGAATTCCGAGAGGAGAGCCAAGCTG (recognition site for restriction enzyme EcoRI is underlined) | 14 |
| Forward primer 2 | CCACCGCCCTCAAAGTAGACG | 15 |
| Reverse primer 2 | CCTCACATTGCCAAAAGACG | 16 |
| Reverse primer 3 | GTCGATGTCATGATCTTTATAATCGAGGGGCCAAGGG CA | 17 |
| Reverse primer 4 | GTCGATGTCATGATCTTTATAATCGTCGATGTCATG | 18 |
| | TCGAATTCCAGCTCATTAGCGCGAGAAC | 19 |
| Forward primer 3 | (recognition site for restriction enzyme EcoRI is underlined) | |
| Reverse primer 5 | CCCTCGAGTTACGTCTCCTCAAATTGTAGATC (recognition site for restriction enzyme XhoI is underlined) | 20 |
| Forward primer 4 | GTTTACGATCACAGTGTCC | 21 |
| Reverse primer 6 | GTCGATGTCATGATCTTTATAATCCGTCTCCTCAAAT TG | 22 |
| Reverse primer 7 | CGAGATCTAGTCCTCTCATTTGCTGG (recognition site for restriction enzyme BglII is underlined) | 23 |

### Example 1

### 1. Preparation of hybridoma producing anti-fPD1 mouse monoclonal antibody

### 1-1 PCR

In order to amplify fPD1 cDNA (nucleotide sequence of NCBI Reference Sequence: NM_001145510.1), PCR was performed with cDNA of a cat lymphoma-derived cell line KO-1 as a template using forward primer 1 (single-stranded DNA consisting of the nucleotide sequence of SEQ ID NO: 13) and reverse primer 1 (single-stranded DNA consisting of the nucleotide sequence of SEQ ID NO: 14), and KOD-Plus-Neo (manufactured by Toyobo Co., Ltd.) in accordance with a protocol attached to the product. The PCR is performed under conditions of pre-denaturation at 94°C for 2 minutes, followed by 35 cycles each involving denaturation at 98°C for 10 seconds, annealing reaction at 58°C for 30 seconds, and elongation reaction at 68°C for 60 seconds, and final elongation reaction was performed at 68°C for 10 minutes. PCR given below was also performed under these conditions.

### 1-2 Sequencing

The PCR product (i.e., fPD1 cDNA) was gel-purified, cleaved with BamHI and EcoRI, and then inserted to between two restriction enzyme sites (BamHI and EcoRI) in pMXs-IP vector to prepare pMX-IP-fPD1. On the basis of this construct vector, PCR was performed using forward primer 2 (single-stranded DNA consisting of the nucleotide sequence of SEQ ID NO: 15) and reverse primer 2 (single-stranded DNA consisting of the nucleotide sequence of SEQ ID NO: 16), and BigDye(R) Termination v3.1 Cycle Sequencing Kit (manufactured by PerkinElmer, Inc.) in accordance with a protocol attached to the product. Then, the amplified PCR product was confirmed to have a nucleotide sequence (cat PD-1 gene consisting of the nucleotide sequence of SEQ ID NO: 12) encoding fPD1 (cat PD-1 consisting of the amino acid sequence of SEQ ID NO: 11), using ABI Prism(R) 377 automatic DNA sequencer (manufactured by Applied Biosystems Inc.).

### 1-3 Preparation of fPD1 expression retrovirus vector

In order to construct a retrovirus vector for expression of a protein of two FLAG tags (2 × FLAG) fused to the C terminus of fPD1 (FLAG-fused fPD1), PCR was first performed with pMX-IP-fPD1 as a template using forward primer 1 and reverse primer 3 (single-stranded DNA consisting of the nucleotide sequence of SEQ ID NO: 17 [i.e., a nucleotide sequence encoding a polypeptide containing the FLAG tag at the C terminus of fPD1]), and KOD-Plus-Neo in accordance with a protocol attached to the product to obtain a primary PCR product. Next, PCR was performed with the primary PCR product as a template using forward primer 1 and reverse primer 4 (single-stranded DNA consisting of the nucleotide sequence of SEQ ID NO: 18 [i.e., a nucleotide sequence encoding the 2 × FLAG tag]), and KOD-Plus-Neo in accordance with a protocol attached to the product to obtain a secondary PCR product. The obtained secondary PCR product was introduced to a restriction enzyme site SmaI of a pbluescript SK(-) vector, then further cleaved with EcoRI and NotI, and then inserted to between two restriction enzyme sites (EcoRI and NotI) in pMXs-IP vector to prepare pMX-IP-fPD1-FL (i.e., a fPD1 expression retrovirus vector).

### 1-4 Preparation of fPD1-expressing mouse cell line

3.5 × 10⁵ cells of an NIH3T3 cell line were inoculated to each well of a 6-well dish. 1 day later, the NIH3T3 cell line was transfected with pMX-IP-fPD1-FL using PEI Max (manufactured by Polysciences, Inc.) in accordance with a protocol attached to the product, and incubated for 48 hours. Then, the cells were cultured in the presence of 10 µg/ml of puromycin (manufactured by Sigma-Aldrich Co., LLC) to prepare NIH3T3/fPD1 (i.e., an NIH3T3 cell line stably expressing fPD1).

Stable expression of fPD1 in the NIH3T3/fPD1 was confirmed by the immunofluorescent method using an anti-FLAG M2 antibody (1:1000 dilution, manufactured by Sigma-Aldrich Co., LLC) as a primary antibody, and IgG-Alexa(R) 647-labeled anti-mouse (manufactured by Jackson Immunoresearch Laboratories Inc.) as a secondary antibody.

### 1-5 Immunization of mouse with NIH3T3/fPD1 and subsequent screening for NIH3T3/fPD1-positive hybridoma

In order to prepare a mouse monoclonal antibody against fPD1, 500 µL of DMEM culture solution containing 1 × 10⁷ cells of NIH3T3/fPD1 was emulsified with an equal amount of Titer Max(R) Gold (manufactured by CytRx Corp.) and intradermally administered to the hind footpad of each 5-week-old Balb/c mouse (manufactured by Japan SLC, Inc.). The administration was performed a total of four times every other week, and 10 days after final administration, popliteal lymph node cells were isolated and fused with P3U1 cells. Specifically, lymph node monocytes (1 × 10⁸ cells) and P3U1 cells (2 × 10⁷ cells) were mixed and washed twice with serum-free RPMI1640 culture solution. Then, the cells were fused in accordance with the instruction of ClonaCell-HY Hybridoma Kit (manufactured by Veritas Corp.). Then, the cells were inoculated to four 96-well tissue culture plates (100 µL/well). Colonies were obtained and then screened for NIH3T3 cells-positive hybridomas expressing fPD1 by ELISA or flow cytometry. As a result, 288 types of NIH3T3/fPD1-positive hybridomas were obtained.

### 1-6 Screening for hybridoma producing anti-fPD1 mouse monoclonal antibody

From among the 288 types of NIH3T3/fPD1-positive hybridomas, hybridomas producing an anti-fPD1 antibody were screened for by ELISA. As a result, four types of hybridomas (hybridoma 1A1, hybridoma 1A11, hybridoma 1D3, and hybridoma 1H11) were obtained (in the present specification, an anti-fPD1 mouse monoclonal antibody produced by the hybridoma 1A1 is also referred to as "1A1", an anti-fPD1 mouse monoclonal antibody produced by the hybridoma 1A11 is also referred to as "1A11", an anti-fPD1 mouse monoclonal antibody produced by the hybridoma 1D3 is also referred to as "1D3", and an anti-fPD1 mouse monoclonal antibody produced by the hybridoma 1H11 is also referred to as "1H11").

### Example 2

### 2. Confirmation that anti-fPD1 mouse monoclonal antibody binds to fPD1

In order to confirm the four types of anti-fPD1 mouse monoclonal antibodies (1A1, 1A11, 1D3, and 1H11) to bind to fPD1 in NIH3T3/fPD1, flow cytometry analysis was conducted in accordance with the method described in the document "Mizuno et al., J Vet Med Sci, 71 (12): 1561-1568, 2009". Specifically, 2 × 10⁵ cells of NIH3T3/fPD1 or NIH3T3 were incubated in the presence of 10 µg/mL each of the four types of mouse monoclonal antibodies, and incubated in the presence of anti-mouse IgG-PE (manufactured by SouthernBiotech), followed by the detection of PE-derived fluorescent brightness using a flow cytometer (CytoFLEX [manufactured by Beckman Coulter, Inc.]). The obtained results were analyzed using FlowJo software (manufactured by Tree Star Inc.).

As a result, in the case of using any of the anti-fPD1 mouse monoclonal antibodies, fluorescence intensity derived from the fluorescently labeled secondary antibody (anti-mouse IgG-PE) was increased (Figure 1).

This result indicates that the four types of anti-fPD1 mouse monoclonal antibodies (1A1, 1A11, 1D3, and 1H11) bind to fPD1 in NIH3T3/fPD1.

### Example 3

### 3. Confirmation that anti-fPD1 mouse monoclonal antibody (1A1) inhibits binding between fPD1 and fPDL1

### 3-1 Construction of fPDL1 expression retrovirus vector

In order to amplify fPDL1 cDNA (nucleotide sequence of NCBI Reference Sequence: XM_006939039.3), PCR was performed with cDNA of a cat lymphoblast line FL-4 as a template using forward primer 3 (single-stranded DNA consisting of the nucleotide sequence of SEQ ID NO: 19) and reverse primer 5 (single-stranded DNA consisting of the nucleotide sequence of SEQ ID NO: 20), and KOD-Plus-Neo (manufactured by Toyobo Co., Ltd.) in accordance with a protocol attached to the product. The amplified PCR product (i.e., fPDL1 cDNA) was gel-purified, cleaved with EcoRI and XhoI, and then inserted to between two restriction enzyme sites (EcoRI and XhoI) in pMXs-IP vector to prepare pMX-IP-fPDL1-K. On the basis of this construct vector, PCR was performed using forward primer 2 (single-stranded DNA consisting of the nucleotide sequence of SEQ ID NO: 15) and reverse primer 2 (single-stranded DNA consisting of the nucleotide sequence of SEQ ID NO: 16), and BigDye(R) Termination v3.1 Cycle Sequencing Kit (manufactured by PerkinElmer, Inc.) in accordance with a protocol attached to the product. Then, the amplified PCR product was confirmed to have a nucleotide sequence (cat PD-L1 gene consisting of the nucleotide sequence of SEQ ID NO: 12) encoding fPDL1 (cat PD-L1 consisting of the amino acid sequence of SEQ ID NO: 11), using ABI Prism(R) 377 automatic DNA sequencer (manufactured by Applied Biosystems Inc.).

### 3-2 Preparation of fPDL1-hIg expression vector

In order to amplify a polynucleotide encoding an extracellular region of fPDL1, PCR was performed with pMx-IP-fPDL1 as a template using forward primer 3 (single-stranded DNA consisting of the nucleotide sequence of SEQ ID NO: 19) and reverse primer 6 (single-stranded DNA consisting of the nucleotide sequence of SEQ ID NO: 22), and KOD-Plus-Neo (manufactured by Toyobo Co., Ltd.) in accordance with a protocol attached to the product to obtain a primary PCR product. Next, PCR was performed with the primary PCR product as a template using forward primer 3 and reverse primer 4 (single-stranded DNA consisting of the nucleotide sequence of SEQ ID NO: 18 [i.e., a nucleotide sequence encoding the 2 × FLAG tag]), and KOD-Plus-Neo in accordance with a protocol attached to the product to obtain a secondary PCR product. The obtained secondary PCR product was inserted to a pANT vector to prepare pANT-fPD1-FL. Next, PCR was performed with pANT-fPD1-FL as a template using forward primer 4 and reverse primer 7 (nucleotide sequence of SEQ ID NO: 23), and KOD-Plus-Neo in accordance with a protocol attached to the product to obtain a tertiary PCR product (i.e., a polynucleotide encoding the extracellular region of fPDL1). The obtained tertiary PCR product was cleaved with BglII and inserted to between two restriction enzyme sites (EcoRV and BgIII) in pFUSE-hIgG2-Fc2 (manufactured by InvivoGen) (i.e., a human IgG2 Fc region [hereinafter, also referred to as "hIg"] expression vector) to prepare pFUSE-fPDL1-hIg (i.e., an fPDL1-hIg expression vector).

### 3-3 Preparation and purification of fPDL1-hIg

fPDL1-hIg or hIg was expressed into a culture solution of an Expi293F cell line using pFUSE-fPDL1-hIg or pFUSE-hIgG2-Fc2 and Expi293 Expression System Kit (manufactured by Thermo Fisher Scientific Inc.). After recovery of a culture supernatant, fPDL1-hIg and hIg were purified by purification using rProtein A agarose (manufactured by GE Healthcare Japan Corp.) and desalting treatment by dialysis. The purification of fPDL1-hIg and hIg was confirmed by SDS-PAGE and Western blotting using anti-human IgG in accordance with conventional methods (Figure 2A).

### 3-4 Binding test between fPD1 and fPDL1-hIg

In order to confirm fPDL1-hIg to bind to fPD1 in NIH3T3/fPD1 in a preliminary test for "Inhibition test of binding between fPD1 and fPDL1-hIg using anti-fPD1 mouse monoclonal antibody" mentioned later, flow cytometry analysis was conducted in accordance with the method described in the document "Mizuno et al., J Vet Med Sci, 71 (12): 1561-1568, 2009". Specifically, 2 × 10⁵ cells of NIH3T3/fPD1 or NIH3T3 were incubated in the presence of varying concentrations (0 µg/mL, 0.16 µg/mL, 0.63 µg/mL, 2.5 µg/mL, or 10 µg/mL) of fPDL1-hIg, and incubated in the presence of anti-human IgG-PE (manufactured by SouthernBiotech), followed by the detection of PE-derived fluorescent brightness using a flow cytometer (CytoFLEX [manufactured by Beckman Coulter, Inc.]). The obtained results were analyzed using FlowJo software (manufactured by Tree Star Inc.).

As a result, no change in anti-human IgG-PE-derived fluorescence intensity was found when NIH3T3 was incubated in the presence of fPDL1-hIg. By contrast, anti-human IgG-PE-derived fluorescence intensity was found to be increased in an fPDL1-hIg concentration-dependent manner when NIH3T3/fPD1 was incubated in the presence of fPDL1-hIg (Figure 2B).

This result indicates that fPDL1-hIg specifically binds to fPD1 in NIH3T3/fPD1.

### 3-5 Inhibition test of binding between fPD1 and fPDL1 using anti-fPD1 mouse monoclonal antibody

In order to analyze whether each anti-fPD1 mouse monoclonal antibody inhibited the binding between fPD1 and fPDL1, flow cytometry analysis was conducted in accordance with the method described in the document "Mizuno et al., J Vet Med Sci, 71 (12): 1561-1568, 2009". Specifically, 2 × 10⁵ cells of NIH3T3/fPD1 were 1) incubated in the presence of 10 µg/mL each of the four types of anti-fPD1 mouse monoclonal antibodies (1A1, 1A11, 1D3, and 1H11) (arrowhead in Figure 3), 2) incubated in the presence of 10 µg/mL of fPDL1-hIg (arrow in Figure 3), or, 3) incubated in the presence of hybridoma culture supernatants containing the four types of anti-fPD1 mouse monoclonal antibodies and 10 µg/mL of fPDL1-hIg (open arrow in Figure 3), and incubated in the presence of anti-human IgG-PE (manufactured by SouthernBiotech), followed by the detection of PE-derived fluorescent brightness using a flow cytometer (CytoFLEX [manufactured by Beckman Coulter, Inc.]). The obtained results were analyzed using FlowJo software (manufactured by Tree Star Inc.).

As a result, anti-human IgG-PE-derived fluorescence intensity increased by the incubation of NIH3T3/fPD1 in the presence of fPDL1-hIg (arrow in Figure 3) was decreased by incubation with two types of anti-fPD1 mouse monoclonal antibodies (1A1 and 1D3) (open arrow in Figures 3A and 3C). By contrast, such decrease was not found as to two types of anti-fPD1 mouse monoclonal antibodies (1A11 and 1H11) (open arrow in Figures 3B and 3D).

This result indicates that among the four types of anti-fPD1 mouse monoclonal antibodies (1A1, 1A11, 1D3, and 1H11), 1A1 and 1D3 have action of inhibiting the binding between fPD1 and fPDL1 in fPDL1-hIg.

### Example 4

### 4. Confirmation that anti-fPD1 mouse monoclonal antibody (1A1-2) inhibits binding between fPD1 and fPDL1

In order to isolate a more highly pure hybridoma producing an anti-fPD1 mouse monoclonal antibody having action of inhibiting the binding between fPD1 and fPDL1 in light of the results of Example 3, two types of hybridomas (hybridoma 1A1 and hybridoma 1D3) were subjected to single-cell cloning in accordance with a conventional method. As a result, three types of hybridomas (hybridoma 1A1-1, hybridoma 1A1-2, and hybridoma 1A1-3) were isolated from the hybridoma 1A1, and three types of hybridomas (hybridoma 1D3-1, hybridoma 1D3-2, and hybridoma 1D3-3) were isolated from the hybridoma 1D3 (in the present specification, a mouse monoclonal antibody produced by the hybridoma 1A1-1 is also referred to as "1A1-1", a mouse monoclonal antibody produced by the hybridoma 1A1-2 is also referred to as "1A1-2", a mouse monoclonal antibody produced by the hybridoma 1A1-3 is also referred to as "1A1-3", a mouse monoclonal antibody produced by the hybridoma 1D3-1 is also referred to as "1D3-1", a mouse monoclonal antibody produced by the hybridoma 1D3-2 is also referred to as "1D3-2", and a mouse monoclonal antibody produced by the hybridoma 1D3-3 is also referred to as "1D3-3").

In order to examine whether these six types of anti-fPD1 mouse monoclonal antibodies (1A1-1, 1A1-2, 1A1-3, 1D3-1, 1D3-2, and 1D3-3) had action of inhibiting the binding between fPD1 and fPDL1, flow cytometry analysis was conducted in accordance with the method described in the above section "3-5".

As a result, five types of anti-fPD1 mouse monoclonal antibodies (1A1-1, 1A1-3, 1D3-1, 1D3-2, and 1D3-3) had no action of inhibiting the binding between fPD1 and fPDL1 (Figures 4A and 4C to 4F), whereas one type anti-fPD1 mouse monoclonal antibody (1A1-2) was confirmed to have action of inhibiting the binding between fPD1 and fPDL1 (Figure 4B). The hybridoma 1A1-2 is kept in store in Joint Faculty of Veterinary Medicine, Yamaguchi University and is available under a certain condition.

### Example 5

### 5. Confirmation that "1A1-2" specifically binds to fPD1

NIH3T3/fPD1 was subjected to Western blotting using an anti-FLAG antibody (M2, manufactured by Sigma-Aldrich Co., LLC) and "1A1-2" in accordance with a conventional method.

As a result, a band of the same size as that of a band detected for the anti-FLAG antibody was also detected for "1A1-2" (Figure 5A).

NIH3T3/fPD1 or NIH3T3 was subjected to flow cytometry analysis using varying concentrations (0 µg/mL, 0.16 µg/mL, 0.63 µg/mL, 2.5 µg/mL, or 10 µg/mL) of "1A1-2" in accordance with the method described in Example 2 above.

As a result, "1A1-2" was found to specifically bind to NIH3T3/fPD1 in a concentration-dependent manner (Figure 5B).

Further, the fPD1 gene was knocked out from a cat lymphoma cell line (FT-1) using the CRISPR/Cas9 system described in the document "Vet Comp Oncol. 2020 Dec; 18 (4): 739-752" to prepare FT-1/ko-fPD1. Then, FT-1/ko-fPD1 or FT-1 was subjected to flow cytometry analysis using 10 µg/mL of "1A1-2" in accordance with the method described in Example 2 above.

As a result, "1A1-2" was found to specifically bind to fPD1 in FT-1 (Figure 5C).

### Example 6

### 6. Confirmation that "1A1-2" inhibits binding between fPD1 and fPDL1

In order to further analyze whether "1A1-2" inhibited the binding between fPD1 and fPDL1, NIH3T3/fPD1 was 1) incubated in the absence of "1A1-2" and in the presence of hIg, 2) incubated in the presence of varying concentrations (0 µg/mL, 0.16 µg/mL, 0.63 µg/mL, 2.5 µg/mL, 10 µg/mL, or 40 µg/mL) of "1A1-2" and fPDL1-hIg, or 3) incubated in the presence of 40 µg/mL of "1A1-2", and incubated in the presence of anti-human IgG-PE, followed by flow cytometry analysis in accordance with the method described in the above section "3-5".

As a result, anti-human IgG-PE-derived fluorescence intensity increased by the incubation of NIH3T3/fPD1 in the presence of fPDL1-hIg (not hIg) was found to be decreased in a "1A1-2" concentration-dependent manner by incubation with "1A1-2" (Figure 6).

This result indicates that "1A1-2" has action of inhibiting the binding between fPD1 and fPDL1 in fPDL1-hIg.

### Example 7

### 7. Confirmation that "1A1-2" has action of enhancing IFNγ production

"1A1-2" had action of inhibiting the binding between fPD1 and fPDL1 and was therefore analyzed for whether to have action of canceling the suppression of T cell activation by the binding between fPD1 and fPDL1 and activating T cells. Specifically, PBMC was separated from three healthy cats (cat 1, cat 2, and cat 3) in accordance with a conventional method. Then, the PBMC was inoculated at 2 × 10⁵ cells per well into a 96-well round-bottom plate, and a pseudo-antibody (mouse IgG1, manufactured by BioLegend, Inc.) or "1A1-2" was added thereto at the same time with addition of 10 µg/mL of ConA. After culture for 72 hours, a culture supernatant was recovered, and fIFNγ in the culture supernatant was measured using Feline IFN-gamma DuoSet ELISA (manufactured by R&D Laboratories, Inc.).

As a result, the concentration of fIFNγ produced into the culture solution was significantly increased by the culture of PBMC in the presence of "1A1-2" as compared with culture in the presence of the pseudo-antibody (Figure 7).

This result indicates that "1A1-2" canceled the suppression of T cell activation by the binding between fPD1 and fPDL1 and activated T cells, resulting in an increased amount of fIFNγ produced.

### Example 8

### 8. Identification of amino acid sequences of heavy chain variable region and light chain variable region of "1A1-2"

Table 2 shows the amino acid sequences of the heavy chain variable region and the light chain variable region of "1A1-2" identified in accordance with a conventional method on the basis of the hybridoma 1A1-2.

**[Table 2]**

| |
|---|
| Amino acid sequence of heavy chain variable region of "1A1-2" (SEQ ID NO: 7) |
| QIQLVQSGPELKKPGETVKISCKASGYTFTDYSIHWVKQAPGKGLKWMGWINTETG EPTYADDFKGRFAFSLETSVSAAFLQINNLKNEDTATYFCARPLFYYYITSPRSMD CWGQGTSVSVSS |
| Amino acid sequence of light chain variable region of "1A1-2" (SEQ ID NO: 8) |
| SIVMTQTPKFLLVSAGDRVTITCKASQSVTNDVAWYQQKPGQSPKLLIYYASNRYT GVPDRFTGSGYGTDFTFTISTVQAEDLAVYFCQQDYSSPYTFGGGTMLEIKRA |

It is known that antibody H chain CDR1 resides at positions H31 to H35 based on the Kabat numbering, antibody H chain CDR2 resides at positions H50 to H52, H52A, and H53 to H65 based on the Kabat numbering, and antibody H chain CDR3 resides at positions H95 to H100, H100A to H100G, H101, and H102 based on the Kabat numbering. It is also known that antibody L chain CDR1 resides at positions L24 to L34 based on the Kabat numbering, antibody L chain CDR2 resides at positions L50 to L56 based on the Kabat numbering, and antibody L chain CDR3 resides at positions L89 to L97 based on the Kabat numbering. In consideration of this point, Table 3 shows the amino acid sequences of heavy chain CDR1 to CDR3 and the amino acid sequences of light chain CDR1 to CDR3 in "1A1-2". These CDRs correspond to the boxed amino acid sequences in Table 2.

**[Table 3]**

| Various CDRs in "1A1-2" | Amino acid sequence |
|---|---|
| Heavy chain CDR1 | DYSIH (SEQ ID NO: 1) |
| Heavy chain CDR2 | WINTETGEPTYADDFKG (SEQ ID NO: 2) |
| Heavy chain CDR3 | PLFYYYITSPRSMDC (SEQ ID NO: 3) |
| Light chain CDR1 | KASQSVTNDVA (SEQ ID NO: 4) |
| Light chain CDR2 | YASNRYT (SEQ ID NO: 5) |
| Light chain CDR3 | QQDYSSPYT (SEQ ID NO: 6) |

### Example 9

### 9. Preparation of chimeric antibody consisting of heavy chain variable region and light chain variable region of "1A1-2", and constant regions of cat IgG1

Polynucleotides encoding the constant regions of "1A1-2", contained in the genomic DNA of the hybridoma 1A1-2, were substituted by polynucleotides encoding cat-derived constant regions through the use of the CRISPR/Cas9 system in accordance with the method described in the document "Sci Adv. 2019 Aug 28; 5 (8): eaaw1822" to prepare a hybridoma ch-1A1-2 producing a chimeric antibody consisting of the heavy chain variable region of "1A1-2" and the constant region of cat IgG1, and the light chain variable region of "1A1-2" and the constant region of cat IgG1 (in the present specification, a mouse monoclonal chimeric antibody produced by the hybridoma ch-1A1-2 is also referred to as "ch-1A1-2"). Table 4 shows the amino acid sequences of the heavy chain and the light chain of "ch-1A1-2". The hybridoma ch-1A1-2 is kept in store in Joint Faculty of Veterinary Medicine, Yamaguchi University and is available under a certain condition.

**[Table 4]**

| |
|---|
| Amino acid sequence of heavy chain of "ch-1A1-2" (SEQ ID NO: 9) |
| QIQLVQSGPELKKPGETVKISCKASGYTFTDYSIHWVKQAPGKGLKWMGWINTETGEPTYAD DFKGRFAFSLETSVSAAFLQINNLKNEDTATYFCARPLFYYYITSPRSMDCWGQGTSVSVSS ASTTAPSVFPLAPSCGTTSGATVALACLVLGYFPEPVTVSWNSGALTSGVHTFPSVLQASGL YSLSSMVTVPSSRWLSDTFTCNVAHPPSNTKVDKTVRKTDHPPGPKPCDCPKCPPPEMLGGP SIFIFPPKPKDTLSISRTPEVTCLVVDLGPDDSDVQITWFVDNTQVYTAKTSPREEQFNSTY RVVSVLPILHQDWLKGKEFKCKVNSKSLPSPIERTISKAKGQPHEPQVYVLPPAQEELSRNK VSVTCLIKSFHPPDIAVEWEITGQPEPENNYRTTPPQLDSDGTYFVYSKLSVDRSHWQRGNT YTCSVSHEALHSHHTQKSLTQSPGK |
| Amino acid sequence of light chain of "ch-1A1-2" (SEQ ID NO: 10) |
| SIVMTQTPKFLLVSAGDRVTITCKASQSVTNDVAWYQQKPGQSPKLLIYYASNRYTGVPDRF TGSGYGTDFTFTISTVQAEDLAVYFCQQDYSSPYTFGGGTMLEIKRSDAQPSVFLFQPSLDE LHTGSASIVCILNDFYPKEVNVKWKVDGVVQNKGIQESTTEQNSKDSTYSLSSTLTMSSTEY QSHEKFSCEVTHKSLASTLVKSFNRSECQRE |

| |
|---|
| In the table, constant regions are underlined. |

### Example 10

### 10. Confirmation that "ch-1A1-2" 1) specifically binds to fPD1 in NIH3T3/fPD1, 2) inhibits the binding between fPD1 and fPDL1, and 3) has action of enhancing IFNγ production

NIH3T3/fPD1 ("fPD1" in the drawing) or NIH3T3 ("mock" in the drawing) was subjected to flow cytometry analysis using varying concentrations (0 µg/mL, 0.16 µg/mL, 0.63 µg/mL, 2.5 µg/mL, or 10 µg/mL) of "ch-1A1-2" in accordance with the method described in Example 2 above except that anti-mouse IgG-PE was changed to anti-cat IgG-PE (manufactured by Santa Cruz Biotechnology, Inc.).

As a result, "ch-1A1-2" was found to specifically bind to NIH3T3/fPD1 in a concentration-dependent manner (Figure 8A).

Next, in order to analyze whether "ch-1A1-2" inhibited the binding between fPD1 and fPDL1, NIH3T3/fPD1 was 1) incubated in the absence of "ch-1A1-2" and in the presence of hIg, or 2) incubated in the presence of varying concentrations (0 µg/mL, 0.16 µg/mL, 0.63 µg/mL, 2.5 µg/mL, 10 µg/mL, or 40 µg/mL) of "ch-1A1-2" and fPDL1-hIg, and incubated in the presence of anti-human IgG-PE, followed by flow cytometry analysis in accordance with the method described in the above section "3-5".

As a result, anti-human IgG-PE-derived fluorescence intensity increased by the incubation of NIH3T3/fPD1 in the presence of fPDL1-hIg (not hIg) was found to be decreased in a "ch-1A1-2" concentration-dependent manner by incubation with "ch-1A1-2" (Figure 6).

This result indicates that "ch-1A1-2" has action of inhibiting the binding between fPD1 and fPDL1 in fPDL1-hIg.

Next, in order to analyze whether "ch-1A1-2" had action of canceling the suppression of T cell activation by the binding between fPD1 and fPDL1 and activating T cells, PBMC was separated from four healthy cats (cat 1, cat 2, cat 3, and cat 4) in accordance with a conventional method. Then, the PBMC was inoculated at 2 × 10⁵ cells per well into a 96-well round-bottom plate, and a pseudo-antibody (cat IgG1, manufactured by Jackson Immunoresearch Laboratories Inc.) or "ch-1A1-2" was added thereto at the same time with addition of 10 µg/mL of ConA. After culture for 72 hours, a culture supernatant was recovered, and fIFNγ in the culture supernatant was measured using Feline IFN-gamma DuoSet ELISA (manufactured by R&D Laboratories, Inc.).

As a result, the concentration of fIFNγ produced into the culture solution was significantly increased by the culture of PBMC in the presence of "ch-1A1-2" as compared with culture in the presence of the pseudo-antibody (Figure 8C).

This result indicates that "ch-1A1-2" canceled the suppression of T cell activation by the binding between fPD1 and fPDL1 and activated T cells, resulting in an increased amount of fIFNγ produced.

### Industrial Applicability

The present invention contributes to immunotherapy (e.g., cancer immunotherapy) for cats.

## Claims

1. An antibody that specifically binds to cat PD-1 consisting of the amino acid sequence represented by SEQ ID NO: 11, the antibody comprising
heavy chain complementarity-determining region (CDR) 1 consisting of the amino acid sequence represented by SEQ ID NO: 1, heavy chain CDR2 consisting of the amino acid sequence represented by SEQ ID NO: 2, and heavy chain CDR3 consisting of the amino acid sequence represented by SEQ ID NO: 3, and
light chain CDR1 consisting of the amino acid sequence represented by SEQ ID NO: 4, light chain CDR2 consisting of the amino acid sequence represented by SEQ ID NO: 5, and light chain CDR3 consisting of the amino acid sequence represented by SEQ ID NO: 6.

2. The antibody according to claim 1, comprising a heavy chain variable region consisting of an amino acid sequence having 80% or higher sequence identity to the amino acid sequence represented by SEQ ID NO: 7, and a light chain variable region consisting of an amino acid sequence having 80% or higher sequence identity to the amino acid sequence represented by SEQ ID NO: 8.

3. The antibody according to claim 1, comprising a heavy chain consisting of an amino acid sequence having 80% or higher sequence identity to the amino acid sequence represented by SEQ ID NO: 9, and a light chain consisting of an amino acid sequence having 80% or higher sequence identity to the amino acid sequence represented by SEQ ID NO: 10.

4. A polynucleotide encoding the antibody according to any one of claims 1 to 3.

5. A vector comprising a promoter and the polynucleotide according to claim 4 operably linked downstream of the promoter.

6. A host cell in which the vector according to claim 5 has been introduced.

7. An agent for inhibiting the binding between cat PD-1 and cat PD-L1, comprising the antibody according to any one of claims 1 to 3.

8. A method for inhibiting the binding between cat PD-1 and cat PD-L1, comprising the step of administering the antibody according to any one of claims 1 to 3 to a cat.

9. A kit for inhibiting the binding between cat PD-1 and cat PD-L1, comprising the antibody according to any one of claims 1 to 3 or a labeled form thereof, or the vector according to claim 5.
